# EUROPEAN PATENT APPLICATION

(11) **EP 0 884 098 A2**
(43) Date of publication of application: **16.12.1998**
(21) Application number: 98303609.6
(22) Date of filing: 08.05.1998
(51) Int. Cl.: B01J 3/00

(54) **Autoclaves**

(30) Priority: 10.06.1997 GB 9711978
(71) Applicant: Smiths Industries Public Limited Company, London, NW11 8DS (GB)
(72) Inventor: Clayton, Patrick, Worthing, West Sussex BN11 5HT (GB)
(74) Representative: Flint, Jonathan McNeill

(57) **Abstract**

The wire 4 of a temperature sensor 3 or the like is lead through an orifice 23 in the door 12 of an autoclave 1 adjacent a mounting aperture 22. A bolt 24 extends through the mounting aperture 22 and its head 25 compresses a rubber sealing washer 26 about the orifice 23 on the inside of the door. A threaded nut 28 engages the other end of the bolt 24 on the outside of the door 12, the nut having a central boss 30 that exposes the outside end of the orifice 23.

## Description

This invention relates to autoclaves of the kind including a chamber having an outer wall and an orifice through which a wire can extend through the wall.

Autoclaves are often tested by temporarily mounting a sensor, such as a temperature sensor, in the autoclave and connecting this to external monitoring equipment. The wire connecting the sensor to the equipment is led out through a pressure-tight seal. Some of the presently used seals are located in relatively inaccessible locations or require an additional fitting and a pipe connection to be attached to the pressure system.

It is an object of the present invention to provide an improved autoclave and system.

According to one aspect of the present invention there is provided an autoclave of the above-specified kind, characterised in that the orifice is formed through a planar region of the wall to one side of a mounting aperture, and that the autoclave includes a sealing assembly including a planar deformable sealing member overlapping the orifice on one side of the wall, a bolt fastened in the mounting aperture, and a first member on the bolt engaging the deformable sealing member such that the sealing member can be compressed sealingly over the orifice with the wire extending through the orifice.

The bolt preferably extends through the mounting aperture and projects from the other side of the wall, the sealing assembly including a second member on the bolt engaging the other side of the wall. The bolt and second member are preferably screw threaded. The second member may have a central boss that exposes the orifice and the first member may be the head of the bolt. The sealing member is preferably located on the inside of the autoclave. The autoclave may have a plurality of orifices spaced around the mounting aperture and the sealing member may be of a rubber material. The sealing assembly is preferably provided on a door of the autoclave.

According to another aspect of the present invention there is provided an autoclave system including an autoclave according to the above one aspect of the invention, a sensor located in the chamber, a wire connected at one end with the sensor and extending through the orifice, and equipment outside the chamber connected with the opposite end of the wire.

An autoclave system according to the present invention will now be described, by way of example, with reference to the accompanying drawing, in which:

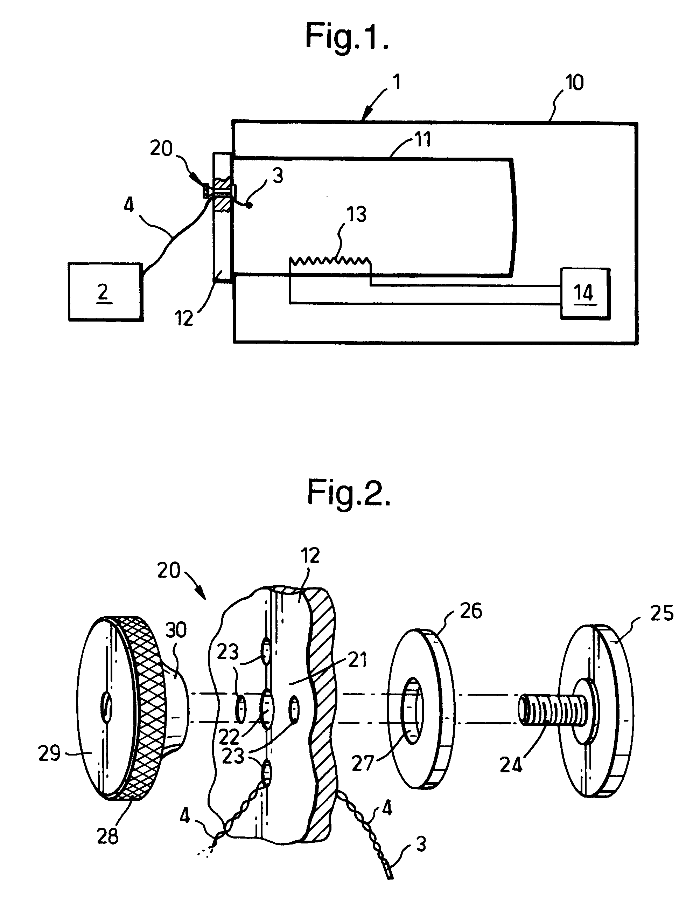

- Figure 1: is a schematic side elevation view of the system; and
- Figure 2: is an exploded perspective view of the sealing assembly.

The autoclave system comprises an autoclave 1, temperature monitoring equipment 2, a thermocouple 3 within the autoclave and a wire 4 connecting the thermocouple with the equipment.

The autoclave 1 has an outer housing 10 containing a pressure chamber 11 of generally cylindrical shape, one end wall of which is provided by a door 12. The chamber 11 has a heater 13 for heating water in the chamber to produce steam at elevated temperature and pressure. The heater 13 is controlled by a control unit 14 within the housing 10, which also controls various other functions of the autoclave such as venting, timing, cooling and the like, in the conventional way.

The door 12 supports a sealing assembly 20, which seals the wire 4 where it extends into the chamber 11. The sealing assembly 20 could be provided on other walls of the chamber 11 but the door 12 is generally most convenient for temporary access. The door 12 has planar region 21 formed with a central aperture 22 and four smaller orifices 23 equally spaced in a circle concentric around the aperture. The sealing assembly 20 includes an internal component in the form of a threaded bolt 24 extending through the aperture 22, the bolt having a first, or internal, clamp member in the form of a circular head 25 located on the inner side of the door 12. The diameter of the head 25 is such that it projects radially outwardly beyond the orifices 23. The bolt 24 carries one or more sealing washers 26 made from a soft, deformable, resilient material such as silicone rubber. The external diameter of the washer 26 is the same as the head 25, the periphery of its central hole 27 being located between the aperture 22 and the orifices 23. The forward surface of the washer 26 lies against the inner surface of the door, its rear surface lying against the forward surface of the head 25.

The sealing assembly 20 is completed by an external clamping component, or second member, in the form of a nut 28 screw threaded onto the end of the bolt 24 where it projects from the forward surface of the door 12. The nut 28 has a knurled disc 29 at its forward end and a tapered boss 30 projecting from the rear side of the disc, the diameter of the boss at its rear end being such that the orifices 23 are exposed around the boss on the forward surface of the door 12.

The wire 4 extends from the equipment 2 through one of the orifices 23 and is bent outwardly to lie between the inner surface of the door 12 and the forward surface of the sealing washer 26. The nut 28 is tightened so that it bears against the forward surface of the door 12 and pulls the head 25 of the bolt 24 forwards to compress the washer 26 on top of the wire 4 and the orifices 23. Pressure produced within the chamber 11 forces the washer 26 more firmly against the inside surface of the door 12, thereby improving the effectiveness of the seal.

Several wires could be fed through the sealing assembly 20, using the different ones of the orifices 23.

The sealing assembly can be installed in the door of an autoclave with little modification since it is only necessary to drill the central aperture 22 and one or more wire-feed orifices 23. The sealing assembly enables ready access for installation and removal of the wire. Although the door is the best location for ease of access, the sealing assembly could be mounted on other walls of the chamber. Where a more permanent feedthrough is needed, it might be preferable for this to be provided through the side or end wall of the chamber, within the outer casing 10. The threaded bolt could be provided on the external component, the internal clamp member having a threaded hole into which the bolt is screwed. Instead of using a threaded bolt, other forms of bolt could be used that enable the sealing washer to be compressed against the inside surface of the wall of the chamber.

## Claims

1. An autoclave including a chamber (11) having an outer wall (12) and an orifice (23) through which a wire (4) can extend through the wall, characterised in that the orifice (23) is formed through a planar region (21) of the wall (12) to one side of a mounting aperture (22), and that the autoclave includes a sealing assembly (20) including a planar deformable sealing member (26) overlapping the orifice (23) on one side of the wall (12), a bolt (24) fastened in the mounting aperture (22), and a first member (25) on the bolt (24) engaging the deformable sealing member (26) such that the sealing member (26) can be compressed sealingly over the orifice (23) with the wire (4) extending through the orifice.

2. An autoclave according to Claim 1, characterised in that the bolt (24) extends through the mounting aperture (22) and projects from the other side of the wall (12), and that the sealing assembly (20) includes a second member (28) on the bolt (24) engaging the other side of the wall (12)

3. An autoclave according to Claim 2, characterised in that the bolt (24) and second member (28) are screw threaded.

4. An autoclave according to Claim 2 or 3, characterised in that the second member (28) has a central boss (30) that exposes the orifice (23).

5. An autoclave according to any one of the preceding claims, characterised in that the first member (25) is a head of the bolt (24).

6. An autoclave according to any one of the preceding claims, characterised in that the sealing member (26) is located on the inside of the autoclave.

7. An autoclave according to any one of the preceding claims including a plurality of orifices (23) spaced around the mounting aperture (22).

8. An autoclave according to any one of the preceding claims, characterised in that the sealing member (26) is of a rubber material.

9. An autoclave according to any one of the preceding claims, characterised in that the sealing assembly (20) is provided on a door (12) of the autoclave.

10. An autoclave system including an autoclave (1) according to any one of the preceding claims, a sensor (3) located in the chamber (11), a wire (4) connected at one end with the sensor (3) and extending through the orifice (23), and equipment (2) outside the chamber (11) connected with the opposite end of the wire.
